# EUROPEAN PATENT APPLICATION

(11) **EP 2 667 196 A1**
(43) Date of publication of application: **27.11.2013**
(21) Application number: 12075052.6
(22) Date of filing: 26.05.2012
(51) Int. Cl.: G01N 33/68

(54) **Bioorthogonal chemical inducers of reversible dimerization for control of protein interactions in cells**

(71) Applicant: Max-Planck-Gesellschaft zur Förderung der Wissenschaften e.V., 80539 München (DE)
(72) Inventor: WU, Yaowen, 44149 Dortmund (DE)
(74) Representative: Arth, Hans-Lothar

(57) **Abstract**

The present application refers to bioorthogonal chemical inducers of reversible dimerization for control of protein interactions in cells. A compound, a test system, methods and uses are disclosed how the invention can be applied in the investigation of intracellular protein interactions. The system is composed of SLF'-TMP as the dimerizer, displaying a high affinity binding to FKBP(F36V) mutant and eDHFR, respectively. The system is reversible on addition of TMP.

## Description

The present application refers to bioorthogonal chemical inducers of reversible dimerization for control of protein interactions in cells.

Chemical inducers of dimerization (CIDs or "dimerizers") have shown to be a powerful tool for modulating protein interactions. They have been widely used in a variety of biological studies in particular in the field of signal transduction.

Methods to perturb and control the intracellular activity and/or localizations of proteins are enormously useful to test a variety of biological processes and cellular networks. Small molecule perturbation has been of great interest, since it can be operated in acute, reversible and tunable fashion. A dimerizer binds simultaneously to two protein modules to form a homodimer or heterodimer. Dimerizers allow for the control of protein-protein interactions and have been shown to be a powerful tool for the investigation of biological events. In these applications, proteins of interest are fused to the binding modules recognized by the respective dimerizer. Treatment with the dimerizer brings fusion proteins together. In this way protein function and thus the cellular events downstream can be modulated in real time.

Rapamycin was the first naturally occurring dimerizer described in literature. Up to now this compound has been extensively analyzed. Rapamycin mediates the heterodimerization of FK506-binding protein (FKBP) and the FKBP-rapamycin-binding (FRB) domain of kinase mTOR (mammalian target of rapamycin) protein. Rapamycin has been broadly used for controlling protein function in many biological processes, including gene transcription, signal transduction, post-translational protein modification and protein degradation. Rapamycin is a natural dimerizer binding to endogenous FKBP and mTOR proteins.

*F*KBPs, or FK506 binding proteins, belong to a protein family with prolyl isomerase activity. Their function is related to cyclophilins. The amino acid sequence, however, is not related. FKBPs have been identified in many eukaryotes from yeast to humans and function as protein folding chaperones for proteins with proline residues. Cyclophilins and FKBPs build the immunophilin family.

The protein mTOR is also known as FK506 binding protein 12-rapamycin associated protein 1 (FRAP1). In humans this protein is encoded by the FRAP1 gene. mTOR is a serine/threonine protein kinase that regulates cell growth, cell proliferation, cell motility, cell survival, protein synthesis, and transcription. mTOR belongs to the phosphatidylinositol 3-kinase-related kinase protein family.

Heterodimerization of FKBP and FRB domain by rapamycin inhibits the kinase activity of mTOR, leading to undesirable biological activities such as immunosuppression and induction of autophagy. This severely restricts its use in certain applications *in vivo.* Moreover, FKBPs are ubiquitous and abundant in eukaryotic cells. Sequestration of compound by endogenous FKBPs could attenuate the efficacy of dimerizer. FKBP-binding ligands such as rapamycin and FK506 have been shown to interfere with cellular functions of FKBP proteins in regulation of intracellular calcium release.

The rapamycin-based dimerizer system was commercialized by ARIAD Pharmaceuticals as the ARGENT™ regulated heterodimerization kit and now by Clontech Laboratories as the iDimerize™ Inducible Heterodimer System. While useful in many cases, conventional rapamycin-based dimerizers have limitations in certain applications *in vivo,* since they bind to endogenous protein targets, thus leading to undesirable physiological activities.

Therefore, to eliminate off-target effects of rapamycin on native mTOR protein, extensive work has been done to make improved rapamycin analogs (rapalogs) that only bind to mutant FRB domain. Likewise, engineering of a synthetic ligand of FKBP (SLF) and FKBP protein using the "bump and hole" principle led to a homodimerizer that is only recognized by the FKBP mutant (F36V). The toolkit of chemically induced dimerization system has been substantially expanded.

However, there remains a high demand for reversible systems for controlling protein function with minimal cellular interference. An ideal CID system would allow for reversibly controlling protein function with minimal intracellular interactions.

Such systems that do not or only to a negligible degree interfere with physiological signal pathways in the cell, in other words are inert to physiological interactions, are called bioorthogonal.

Therefore it is the task of the present invention to provide a dimerizer system that allows for forming homodimers or heterodimers of any intracellular peptidic compound and/or similar exogenous compound brought into a cell, preferably an eukaryotic cell, that enables testing their respective intracellular interactions without activating or biasing other intracellular signal pathways. Ideally, the inventive dimerizer should work as a bioorthogonal system.

Surprisingly, this problem can be solved by the compounds, uses and methods disclosed in the independent claims of the present application. Further advantageous embodiments and applications can be found in the dependent claims, the description and the figures.

The inventive dimerizers are compounds of the general formula (I):

**SLF'** ― **X ― TMP**

Herein, SLF' is an SLF analog, TMP represents trimethoprim and X is a linker molecule selected from the group comprising or consisting of polyethylene glycol (PEG), triethylene glycol, tetraethylene glycol (TEG), alkyl chain with up to 30 carbon atoms, phosphodiesters, glycosides, amides, esters, diesters, thioesters, aldol products, acetate moieties, polyethylenes, isoprenoids, phenyl groups, aromatic groups, hetrocyclic groups, ethers, thioethers, and imines.

As described above, SLF (synthetic ligand of FKBP) is a ligand that binds to the naturally occurring ubiquitous protein FKBP12. The problem of SLF is that it may also bind to other intracellular compounds, especially to further members of the highly conserved FKBP protein family, or other intracellular compounds may act as a ligand with a certain affinity to the FKBP12 binding domain. This may cause a plethora of undesired side effects, a.o. the induction of immunosuppression. This makes the whole system hard to control. It is near to impossible to discern properly which observed effects have been induced by the test compound and which arise from these side effects. SLF has the following structure:

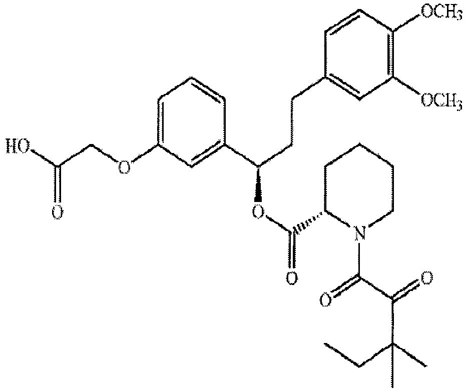

SLF synthesis was described thoroughly by Amara et al. (PNAS 1997, 94, 10618-10623) and Keenan et al. (Bioorg Med Chem. 1998, 6, 309-335).

To overcome this problem a bioorthogonal solution has been found. The SLF analog SLF' binds with high specificity (K_{d} = 0.094 nM) to the binding domain of the FKBP12 mutant FKBP(F36V). This indication means that in this mutant a phenylalanine is replaced by a valine at position 36, counting from the N-terminal end. Besides altered binding characteristics this mutation is silent. The term "FKBP(F36V) is used herein to refer also to a FKBP12 mutant wherein the amino acid replacement is present in an amino acid position corresponding to amino acid position 36 of the wild type sequence of FKBP12. SLF' does not bind at all to naturally occurring (= unmutated) FKBP. SLF' has the following structure:

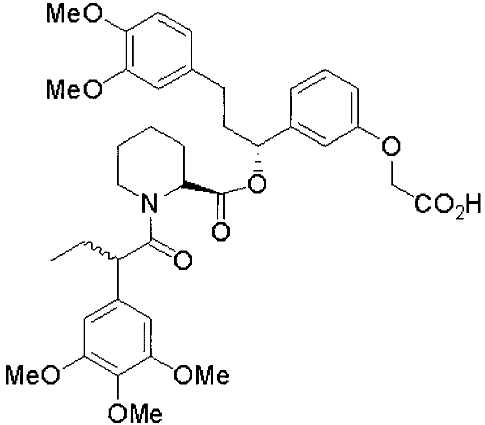

A detailed description of the synthesis of SLF' (compound 7,8) is given in Example 1.

The high affinity to the binding domain of FKBP(F36V) is caused by a different substituent (1-[1-(3,4,5-trimethoxybenzyl)]-propyl) bound to the amide moiety which is formed by the piperidine ring. SLF' shows a very low affinity to non-mutated FKBP and is therefore bioorthogonal. In contrast, SLF alone displays a low affinity to FKBP(F36V). In consequence, SLF' and the binding domain of FKBP(F36V) form a highly specific bump-and-hole molecule. No physiological compound interferes with this system.

On the other end of the compound of general formula (I) is trimethoprim (TMP). TMP (5-(3,4,5-trimethoxybenzyl)pyrimidine-2,4-diamine) is a well-known bacteriostatic antibiotic which is used above all in the prophylaxis and treatment of urinary tract infections. It belongs to the group of dihydrofolate reductase (DHFR) inhibitors. The action of TMP could also be replaced by other DHFR binding ligands, such as methotrexate (MTX).

The current ligand-ligand binding domain (LBD) pairs, SLF'-FKBP(F36V) and TMP-DHFR, could be replaced by other ligand-LBD pairs, which can be organized in a similar way as did in the current invention. For example, the TMP-DHFR pair could be replaced by a selective inhibitor and its cognate mutant DHFR.

TMP has the following structure:

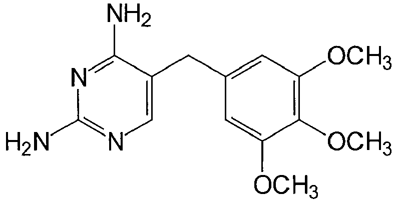

MTX has the following structure:

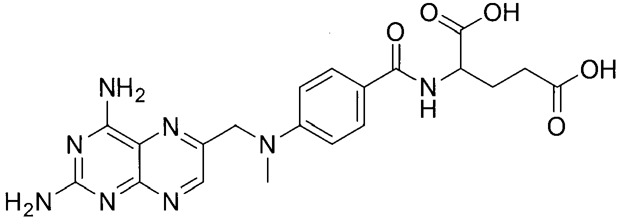

In this construct it is taken advantage of that TMP binds with a high affinity to bacterial (prokaryotic) dihydrofolate reductase (K_{I} = 1 nM), but only with a very low affinity to eukaryotic dihydrofolate reductase (K_{I} = 4-8 µM in mammalian cells). Therefore in the present invention at least the binding domain of a bacterial DHFR is used as a fusion protein moiety fused to a test compound. Its binding partner in a preferred system according to the invention thus is dihydrofolate reductase from *Escherichia coli* (eDHFR). Therefore at least the binding domain of eDHFR is used as the second fusion protein moiety (beside the test compound) in this system. Likewise here there is a bump-and-hole principle. TMP is bioorthogonal to eukaryotic DHFR, and there is no natural ligand for eDHFR in eukaryotic cells. Thus this interaction of TMP and eDHFR is equally highly specific like that of SLF' and FKBP(F36V). No physiological compound interferes with these two interacting systems.

The highly specific binding of SLF' with FKBP(F36V) and of TMP with eDHFR is demonstrated in Example 2.

Thus the present invention refers to a compound of the general formula (I):

**SLF' ― X ― TMP**

wherein X is a linker molecule selected from the group comprising or consisting of polyethylene glycol (PEG), triethylene glycol, tetraethylene glycol (TEG), alkyl chain with up to 30 carbon atoms, phosphodiesters, glycosides, amides, esters, diesters, thioesters, aldol products, acetate moieties, polyethylenes, isoprenoids, phenyl groups, aromatic groups, hetrocyclic groups, ethers, thioethers, and imines. and wherein TMP represents trimethoprim.

In a particularly preferred embodiment the linker molecule X is TEG.

According to the invention SLF' and TMP are connected by a linker molecule. The role of this linker molecule first is to keep these two interacting systems spatially together in order to investigate the physiological interactions of the binding partners of FKBP(F36V) and the bacterial DHFR, in particular eDHFR. On the other hand the linker molecule must ensure an optimal distance so that both binding partners will have the steric chance of an interaction. A further requisite of the linker is that it should enable or at least support the passage of the entire compound of general formula (I) through the cell membrane of the cells of the aqueous test system. Therefore it is desirable that the linker molecule contributes significantly to the lipophilic nature of the entire compound.

The preferred distance between SLF' and TMP to be kept by the linker molecule corresponds to a C2 ― C30 alkyl chain as a backbone of the linker molecule, more preferred to a C5 ― C20 alkyl chain and particularly preferred to a C7 ― C15 alkyl chain.

Suitable linker molecules are but are not limited to: polyethylene glycol (PEG), triethylene glycol, tetraethylene glycol (TEG), alkyl chain with up to 30 carbon atoms, phosphodiesters, glycosides, amides, esters, diesters, thioesters, aldol products, acetate moieties, polyethylenes, isoprenoids, phenyl groups, aromatic groups, hetrocyclic groups, ethers, thioethers, and imines. The most favorite linker is tetraethylene glycol.

The linker molecule must be reacted at both its ends with the non-binding moieties of SLF' and TMP in order to build a stable covalent bond, respectively. The bonding reaction can be selected under peptidic (amide) bonding, ester, thioester, ether, thioether, imine, carbonate, urea, carbamate, ethioate, carbonothioate, guanidine, carbonodithioate, carbamothioate, triazole and bonds formed through cycloaddition, Diels―Alder ligation, or click chemistry.

A particularly preferred linker molecule is tetraethylene glycol bonded at both its ends via an amide bond. Throughout the description, the examples and the figures a preferred molecule comprising tetraethylene glycol bonded at both ends via an amide bond is also referred to as SLF'-TMP.

Therefore a particularly preferred embodiment of the compound of general formula (I) has the following structure:

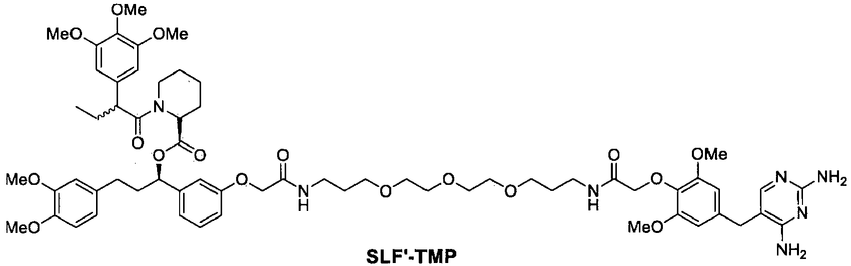

A detailed description of the synthesis of this compound (compound 13) of general formula (I) is given in Example 1.

It is also possible to connect the linker to the oxygen at position C₃ or at position C₅ of the dimethoxyphenyl residue. That means the linker could be bond to the oxygen atom of one of the two methoxy groups on C₃ and C₅. It is not necessary as shown in the SLF'-TMP molecule above that the linker is connected to the oxygen atom at C₄ position. Binding of the linker to the oxygen atom at C₃ or C₅ does not decrease the activity of the obtained molecule.

Fusion proteins or chimeric proteins are proteins created through the joining of two or more DNA-sequences, mostly created through genetic engineering, which originally coded for separate proteins or protein domains. Fusion proteins of at least the binding domains of FKBP(F36V) and a bacterial DHFR, in particular eDHFR, respectively, with the two proteins the interactions of which shall be tested can be generated according to conventional methods. The DNA constructs, respectively vectors coding for the fusion proteins are going to be transfected to the test cell culture by conventional methods.

In a preferred embodiment there is an expression control component included in these DNA constructs in order to detect easily whether the transfection was successful and to which extent. Suitable gene transcription markers include a.o. LacZ-β-galactosidases, luciferase, antibiotic resistant β-lactamases, yeast markers, TetR (tetracycline resistance), KanR (kanamycin resistance), Cm (chloroamphenicol resistance), aada (spectinomycin resistance), araBAD, UR.43, and PLV.

In another preferred embodiment these DNA constructs are going to be expressed under the control of a suitable promoter gene sequence. For example, the cytomegalovirus (CMV) promoter is one of the most commonly used promoters for expression of transgenes in mammalian cells. Ideally, the promoter can be easily regulated by the experimenter. Suitable promoter systems include for example tetracycline controlled promoter systems. It is therefore preferred if the DNA constructs include inducible promoter systems that are regulated by particular chemical or physical factors.

Thus the present invention also refers to a bioorthogonal system for testing intracellular protein interaction in eukaryotic cells, comprising
a) a compound according to claim 1;
b) fusion protein 1 generated from a test compound 1 and at least the SLF' binding domain of FKBP(F36V); and
c) fusion protein 2 generated from a test component 2 and at least the TMP binding domain of a bacterial DHFR.

In this bioorthogonal system test compound 1 and test compound 2 are selected independently from one another among gene products, proteins, protein domains, peptides, polypeptides, glycopeptides, proteins with secondarily modified amino acids, peptides or proteins with protecting groups, saccharides, small molecules, lipids, polynucleotides, oligonucleic acids like DNA or RNA.

In a particularly preferred embodiment the bacterial DHFR of the inventive bioorthogonal system is eDHFR.

The test system for the interaction of the two binding partners, preferably proteins to be tested fused to at least the binding domains of FKBP(F36V) and a bacterial DHFR, in particular eDHFR, respectively, can be interrupted by adding a suitable concentration of DHFR ligand or FKBP(36V) ligand or a combination of both to the test solution. The ligand, preferred TMP will penetrate likewise the cell membrane and will then be competitive with the inventive SLF' ― X ― TMP of general formula (I) at the binding site of a bacterial DHFR, in particular eDHFR. With a sufficiently high TMP concentration SLF' ― X ― TMP will be substantially washed out of this binding. Consequently, the two binding partners test system is efficiently interrupted. Thus this system allows the experimenter to turn on and off the interactions of the test compounds *ad libitum.*

The present invention refers also to a bioorthogonal system as described above, additionally comprising
d) TMP, SLF', or other DHFR or FKBP(36V) ligands such as MTX.

Further, the present invention refers also to a bioorthogonal system with the above-mentioned components a) ― c) or a) ― d), wherein X in SLF' ― X ― TMP is TEG.

Further, the present invention refers also to a bioorthogonal system with the above-mentioned components a) ― c) or a) ― d), wherein test compound 1 or test compound 2 is a fluorescent protein.

Suitable fluorescent proteins can be selected from variants of GFP (green fluorescent protein), including BFP (blue fluorescent protein), ECFP (enhanced cyan fluorescent protein), YFP (yellow fluorescent protein), Citrine (monomeric yellow fluorescent protein variant), RFP (red fluorescence protein), mCherry (monomeric variant of red fluorescence protein), and EGFP (enhanced green fluorescent protein).

The present invention is also directed at the use of any of the aforementioned bioorthogonal systems for testing the interactions of a test compound 1 with a test compound 2.

In a preferred embodiment at least one of test compound 1 and test compound 2 is an intracellular peptidic compound. Such an intracellular peptidic compound can be selected from any protein or peptide expressed physiologically or after transfection inside the cells to be tested, or physiological or artificial derivatives of peptides and proteins such as lipoproteins, glycoproteins, glycopeptides, peptides or proteins with secondarily modified amino acids, peptides or proteins with protecting groups, quaternary structures of proteins, and compounds containing an amino acid chain of at least two amino acids.

These peptidic compounds can contain all physiologically occurring or artificial D- and/or L-amino acids.

As used herein, the term peptide or protein shall also refer to salts, deprotected form, acetylated form of the peptide or protein, deacetylated form of the peptide or protein, enantiomers, diastereomers, racemates, and hydrates peptides and proteins. Diastereomers of the peptide or protein are obtained when the stereochemical or chiral center of one or more amino acids is changed. The enantiomer has the opposite stereochemistry at all chiral centers.

In a preferred embodiment test compound 1 and/or test compound 2 are gene regulating proteins. They may interact with the same gene or different genes.

In a further preferred embodiment test compound 1 or test compound 2 is a pharmaceutical drug. Preferably, this pharmaceutical drug is able to be bonded directly or indirectly to the respective fusion protein moiety using standard methods.

According to the invention a method for testing intracellular protein interaction in eukaryotic cells is disclosed, comprising the following steps:
a) Providing, transfecting and expressing the DNA sequence of a fusion protein 1 from a test component 1 to be tested and at least the SLF' binding domain of FKBP(F36V);
b) providing, transfecting and expressing the DNA sequence of a fusion protein 2 from a test component 2 to be tested and at least the TMP binding domain of a bacterial DHFR;
c) adding SLF' ― X ― TMP according to claim 1 or 2 to cells, preferably eukaryotic cells, and letting them pass the plasma membrane;
d) measuring the physiological effect by determining the change in a selected test parameter system.

As said before, the interaction introduced by a compound according to formula (I) is reversible by addition of a sufficient amount of TMP to the test solution. Therefore the present invention refers also to above said method, wherein the generated effect is reverted by contacting the cells, preferably eukaryotic cells, with TMP in a concentration that allows for competitive replacement of SLF' ― X ― TMP from a bacterial DHFR binding site.

In this method test compound 1 and test compound 2 are selected independently from one another among proteins, protein domains, peptides, polypeptides, glycopeptides, proteins with secondarily modified amino acids, peptides or proteins with protecting groups, saccharides, small molecules, lipids, oligonucleic acids like DNA or RNA.

In a particularly preferred embodiment the bacterial DHFR in this method is eDHFR.

The present application is also directed to this method, wherein test compound 1 and test compound 2 is a physiological peptidic compound, respectively.

A particularly interesting application is to investigate the interactions between a gene regulating protein and a physiological peptidic compound. Therefore the inventive method can be used in an arrangement, wherein test compound 1 is a gene regulating protein and test compound 2 is a physiological peptidic compound, or test compound 1 is a physiological peptidic compound and test compound 2 is gene regulating protein. Such interactions could be investigated in a reversible manner.

Further, an interesting application is to investigate the interactions between two proteins which are part of a complex or need a cofactor for binding under physiological conditions.

Further the CID system may be used to reversibly activate or induce a signaling pathway the physiological stimulus is so far not known or to activate a physiological reaction by dimerization of two proteins or protein domains whenever the experimenter applies a compound according to formula (I). The induced singling pathway could be switched off by adding the competitive ligand (component d).

A useful application of the CID system is to reversibly translocate a physiological peptidic compound in cells. Therefore the inventive method can be used in an arrangement, wherein test compound 1 is a peptide or protein targeting a specific cellular compartment and test compound 2 is a physiological peptidic compound, or test compound 1 is a physiological peptidic compound and test compound 2 is a peptide or protein targeting a specific cellular compartment. The physiological peptidic compound could be relocated to a specific cellular compartment by dimerization whenever the experimenter applies a compound according to formula (I). Subsequent dissociation from the compartment could be achieved by addition of the competitive ligand (component d).

A likewise interesting application is to test the interactions between a pharmaceutical drug (a pharmaceutical drug authorized for medical use in humans and/or animals, a corresponding prodrug or a drug candidate under investigation) and a physiological peptidic compound. Thus the inventive method can be used in an arrangement, wherein test compound 1 is a pharmaceutical drug and test compound 2 is a physiological peptidic compound, or test compound 1 is a physiological peptidic compound and test compound 2 is a pharmaceutical drug.

This test arrangement can be varied by investigating a gene regulating protein and a pharmaceutical drug. Thus the inventive method can be carried out in such a way that test compound 1 is a gene regulating protein and test compound 2 is a pharmaceutical drug, or test compound 1 is a pharmaceutical drug and test compound 2 is a gene regulating protein.

Suitable test parameter systems may be selected depending on the test compounds. Test parameter systems may be selected from the group comprising or consisting of: measurement of an enzymatic reaction like phosphorylation or dephosphorylation, proteolysis, ubiquitination or deubiquitination, glycosylation, acetylation or deacetylation, determining the transcription or translation of a gene of interest or a reporter gene, determining the activation of a signaling pathway, microscopic measurement of protein or peptide localization in cells, dimerization (binding) assay by fluorescent resonance energy transfer (FRET), bioluminescence resonance energy transfer (BRET), protein compliment assay (PCA), fluorescence anisotropy, fluorescence correlation spectroscopy (FCS) / fluorescence cross-correlation spectroscopy (FCCS), yeast two-hybrid (Y2H), size exclusion chromatography, co-sedimentation, pull-down, microarray, isothermal titration calorimetry (ITC), surface plasmon resonance (SPR), microscale thermophoresis, chemical field-effect transistors. The transcription of a transporter gene may be determined by measurement of the corresponding mRNA or of the protein the gene encodes for. Alternatively, the result of an enzymatic reaction may be determined if the gene of interest or the reporter gene encodes for an enzyme.

In molecular biology the use of commercially available kits is becoming ever the more popular. They include all components needed for performing a specific test and/or analysis arrangement in reasonable amounts. Often some or all of the components are already dissolved in a suitable test solution or buffer in useful concentrations.

Therefore this invention also applies to a kit, comprising
a) SLF' ― X ― TMP,
b) TMP, SLF' or other DHFR or FKBP(F36V) ligands such as MTX, and
c) the nucleotide sequences or the vectors including the nucleotide sequences coding for at least the binding domains of FKBP(F36V) and a bacterial DHFR.

Optionally, the kit according to the invention can also comprise
d) chemical means for promoting transfection

These means depend on the transfection method to be used. Possible chemical transfection methods include calcium phosphate method, dendrimer method, lipofection, and polycation-based methods.

It is understood that the transfection step of the inventive methods can also be carried out with non-chemical methods such as electroporation, optical transfection, gene gun using nanoparticles etc.

The inventive methods are particularly useful for a complex as well as variable experimental design. Also after starting an experiment the experimenter could decide to turn off the interaction. Therefore, cellular events initiated by inducing dimerization and disrupting dimerization could be investigated. To our knowledge, no other test system offers at the same time such a high specificity of the reaction combined with a maximum of flexibility.

### Figures:

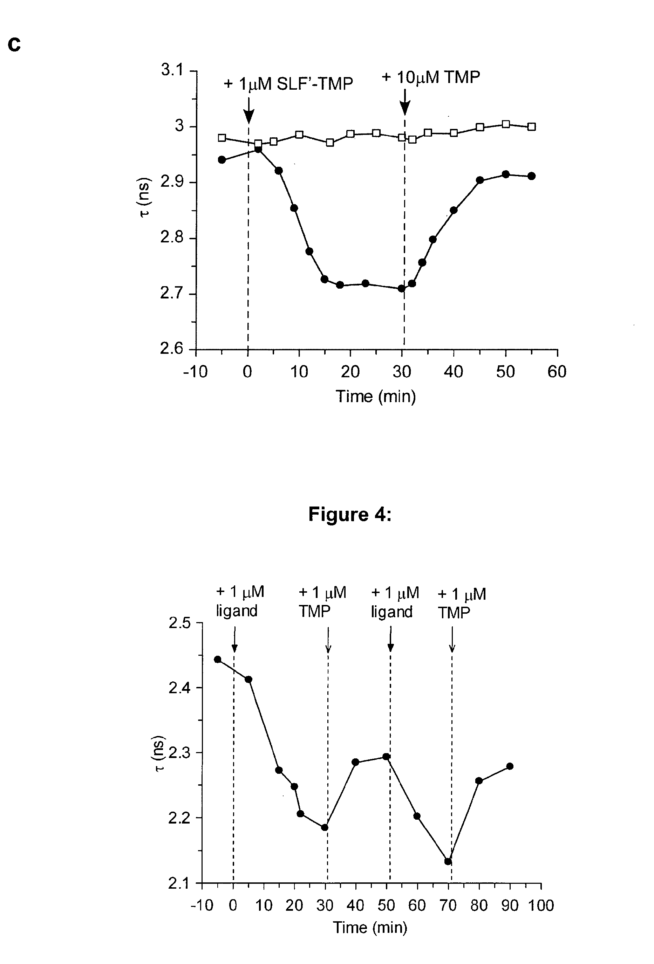

- Figure 1:: Efficiency and specificity of SLF'-TMP binding (comp. example 2). In the absence of SLF'-TMP (solid line) or in the presence of 100 µM SLF'-TMP (dashed line).
a) size exclusion chromatography profile
b) SDS-PAGE analysis
- Figure 2:: a) FRET spectrum of ligand-induced heterodimerization. In the absence of SLF'-TMP (solid line) or in the presence of 400 µM SLF'-TMP (dashed line).
b) Determination of the dissociation constant K_{d}.
- Figure 3:: Chemically induced heterodimerization in cells by FLIM measurements.
a) Confocal microscopy images
b) Reversible heterodimerization in Citrine lifetime images induced by SLF'-TMP and the competitor TMP
c) Response of the average lifetime with 1 µM SLF'-TMP and with subsequent addition of 10 µM competitor TMP in HeLa cells coexpressing Citrine-eDHFR-PM and Cherry-FKBP' (solid circle) and in HeLa cells coexpressing Citrine-eDHFR-PM and Cherry (open square) as a control. PM: a plasma membrane targeting sequence
- Figure 4:: Oscillation of induced protein dimerization with 1 µM SLF'-TMP and 1 µM competitor TMP in HeLa cells coexpressing EGFP-eDHFR-PM and Cherry-FKBP'.

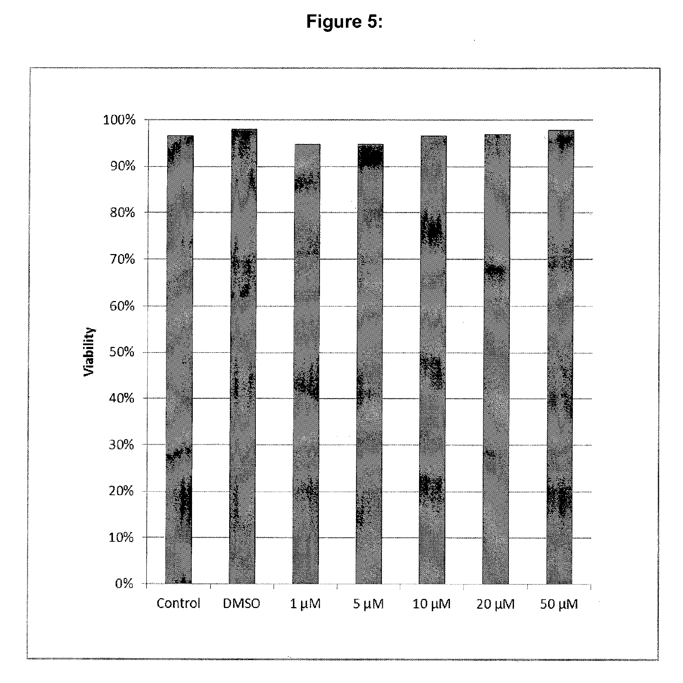

- Figure 5:: Cytotoxicity of SLF'-TMP towards COS-7 cells. 1×10⁵ cells were seeded in each well in 6-well plates. Cells were incubated with indicated amount of ligands or DMSO overnight. And cells were stained by trypan blue and counted by Vi-Cell XR 2.01.

### Examples

### Example 1:

### Synthesis of SLF'-tetraethylene glycol-TMP

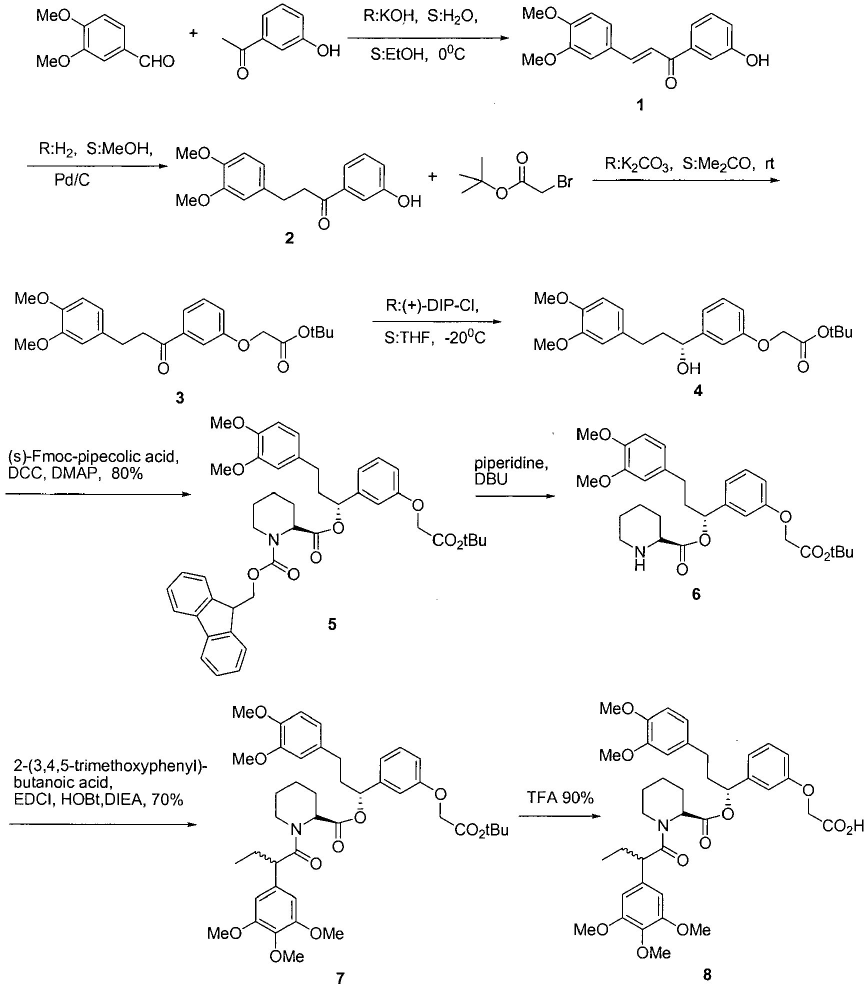

### compound(1): (E)- 3-(3,4-Dimethoxyphenyl)-1-(3-hydroxyphenyl)prop-2-en-1-one

A solution of 3,4-dimethoxybenzaldegyde(60mmol,10 g) and 3'-hydroxyacetophenone (60 mmol, 8.17g) in EtOH (50 mL) was cooled to 0 °C and treated with a cold (10°C) solution of aqueous KOH (100 mL, 240 mmol,13.4 g).The resulting solution was allowed to warm to room temperature and stirred for 24 h at room temperature. The resulting slurry was then poured onto ice containing 12N HCl resulting in a yellowish precipitate (pH=1). The aqueous portion was decanted and the solids triturated with water (3 _{*} 50 mL), then dissolved in EtOAc (100 mL). The organic extract was washed with brine (2 _{*} 200 mL), dried over Na₂SO₄, filtered and concentrated to afford chalcone product 1 (13.8 g, 76%). **TLC** (EtOAc/hexane, 1/1) Rf = 0.4; **¹H NMR** (400 MHz, DMSO-d6 ) δ = 9.76 (s, 1 H), 7.70 (d, J = 6.8 Hz, 2H), 7.61 (d, J = 8 Hz, 1 H), 7.52 (d, J=1.5 Hz, 1H), 7.45 (d, J=1.5 Hz, 1 H), 7.39-7.34 (m, 2H), 7.06-7.00 (m, 2H), 3.85 (s, 3H), 3.81 (s, 3H); **¹³C NMR** (101 MHz, DMSO-d6) δ = 190.0, 158.8, 152.5, 149.9, 145.4, 140.2, 130.7, 128.6, 124.9, 121.0, 120.5, 119.7, 115.8, 112.3, 111.8, 56.5, 56.4 ppm; **ESI-MS** m/z = 285.33 [M+H]⁺.

### compound(2): 3-(3,4-Dimethoxyphenyl)-1-(3-hydroxyphenyl)-1-propanone

A solution of chalcone (6mmol, 1.8g) and Pd/C (10% Pd) in MeOH (100 mL) was hydrogenated wiht H₂ in a Parrhydrogenator for 4 h. The reaction mixture was then filtered and concentrated to a solid. Recrystallization from EtOAc afforded product (1.3 g, 72%) as a colorless solid: TLC (EtOAc/hexane, 1/1) Rf = 0.5; **¹H NMR** (400 MHz, DMSO-d6) 9.74 (s, 1 H), 7.41 (d, J = 8.0 Hz, 1 H), 7.32-7.27 (m, 2H), 7.05 (dd, J = 7.8, 2.1 Hz, 1 H), 6.85-6.71 (m, 3H), 3.71 (s, 3H), 3.68 (s, 3H), 3.25 (t,J = 7.8 Hz, 2H), 2.75 (t, J = 7.2 Hz, 2H); **¹³C NMR** (101 MHz, DMSO-d6) 199.9, 158.2, 149.2, 147.3, 138.0, 133.9, 130.4, 120.7, 119.5, 114.8, 112.6, 112.0, 56.0, 55.9,40.6, 30.0; **ESI-MS** m/z = 287.45 [M+H]⁺.

### compound(3): 1,1-Dimethylethyl [3-[3-(3,4-dimethoxyphenyl)-1-carbonylpropyl] phenoxy]-acetate

A solution of phenol 2 (4.8 mmol, 1.4g) and K₂CO₃ (1.1 g, 8mmol) in acetone (25 mL) was treated with tert-butyl bromoacetate (0.8 mL, 5.4 mmol) and allowed to stir at room temperature for 20 h. After this time the reaction mixture was filtered, concentrated, and flash chromatographed (1:10-1:5-1:3 EtOAc/hexane) to afford product (1.3g, 68%). **TLC** (EtOAc/hexane, 1/1) Rf = 0.8, **¹H NMR** (400 MHz, DMSO-d6 ) 7.55 (d,J = 7.4 Hz, 1H), 7.44 (d, J = 2.1 Hz, 1 H), 7.37 (t, J = 7.8 Hz, 1 H), 7.11 (dd, J = 7.8, 2.1 Hz, 1 H), 6.81-6.74 (m, 3H), 4.54 (s, 2H), 3.85 (s, 3H), 3.83 (s, 3H), 3.24 (t, J = 8.0 Hz, 2H), 3.05 (t, J = 8.0 Hz, 2H), 1.51 (s, 9H); **¹³C NMR** (101 MHz, DMSO-d6) 199.8, 168.3, 158.5, 149.2, 148.1, 138.4, 134.0, 129.9, 121.7, 120.5, 113.4, 112.2, 120.0, 83.2, 66.4, 56.6, 56.4, 41.3, 30.4, 28.2; **ESI-MS** m/z = 423.47 [M+Na]⁺.

### compound(4): (R)-1,1-Dimethylethyl [3-[3-(3,4-dimethoxyphenyl)-1-hydroxypropyl]-phenoxy]-acetate

A solution of ketone 3 (0.87 mmol, 349 mg) in dry THF (10 mL) at -20°C was treated with a solution of (+)-DIP-Chloride (2 mmol,642mg) in THF (10 mL) at -20°C. The resulting mixture was allowed to stand in a -10°C freezer for 24 h after which time the mixture was concentrated and treated with diethylether (10mL) followed by diethanolamine (0.9 mL, 8.7 mmol). The viscous mixture was allowed to stir at room temperature for 6 h after which time it was filtered through a pad of Celite with the aid of EtOAc. The filtrate was concentrated and the crude material flash-chromatographed (30%, then 50% EtOAc/hexane) to afford product (208 mg, 60%). **TLC** (EtOAc/hexane, 1/1) Rf = 0.65, **¹H NMR** (400 MHz, DMSO-d6) 7.26-7.22 (m, 1 H), 6.96-6.92 (m, 2H), 6.80-6.70 (m, 4H), 4.70-4.62 (m, 1 H), 4.52 (s, 2H), 3.88 (s, 3H), 3.87 (s, 3H), 2.68-2.60 (m, 2H), 2.12-1.95 (m, 2H), 1.47 (s, 9H); **¹³C NMR** (101 MHz, DMSO-d6) 168.8, 158.8, 149.1, 147.5, 146.7, 134.5, 129.8, 120.3, 119.4, 114.1, 112.5, 112.1, 111.6, 82.6, 74.2, 66.2, 56.4, 56.1, 41.2, 32.2, 28.1; **ESI-MS** m/z = 425.20 [M+Na]⁺.

### compound(5): (1R)-3-(3,4-Dimethoxyphenyl)-1-[3-(t-butoxycarbonylmethoxy)phenyl]-1-propyl(2S)-1-(9-fluorenylmethoxycarbonyl)-2-piperidine-carboxylate.

A solution of 4 (2.48 mmol, 1 g) in CH₂Cl₂ (5 mL) was treated with N-Fmoc-L-pipecolic acid (3.0 mmol, 850 mg) followed by 1,3-dicyclohexyl carbodiimide (DCC, 620 mg, 3.0 mmol) and 4-(dimethylamino)pyridine (DMAP, 35 mg, 0.3 mmol) under argon atmosphere. The resulting white suspension was allowed to stir overnight at ambient temperature. The reaction mixture was then filtered, evaporated, and flash-chromatographed (silica gel, 10 to 30% EtOAc/hexanes) to afford 1.4g (80%) of the Fmoc-protected pipecolyl ester as a white foam: **TLC** (EtOAc/hexane, 1/2) Rf = 0.60, **¹H NMR** (400 MHz, DMSO-d6) 7.77-7.69 (m, 2H), 7.59 (t, J = 6.8 Hz, 1 H), 7.50-7.15 (m, 6H), 6.95 (d, J = 7.4 Hz, 1 H), 6.86 (s, 1 H), 6.81-6.71 (m, 2H), 6.63-6.58 (m, 2H), 5.77 (br s, 1 H), 5.03 (d, J = 3.5 Hz, 1 H), 4.50-4.23 (m, 5H), 4.16-4.06 (m, 1 H), 3.85 (s, 6H), 3.16 (t, J = 10.8 Hz, 1 H), 2.45-2.55 (m, 2H), 2.34-2.22 (m, 2H), 2.08-2.01 (m, 1 H), 1.79-1.68 (m, 4H), 1.45 (s, 9H), 1.55-1.40 (m, 1 H); **¹³C NMR** (101 MHz, DMSO-d6) 174.3, 171.2, 168.2, 158.5, 147.6, 144.4, 142.2, 133.8, 130.1, 128.2, 127.6, 125.5, 120.6, 120.6, 114.5, 113.9, 112.4, 111.5, 82.5, 76.7, 76.6, 68.1, 66.3, 56.5, 56.4, 47.2, 38.5, 31.8, 31.6, 28.3, 27.3, 25.3, 21.4; **LCMS**(C4, ESI-MS) rt = 10.68 min, m/z = 752.92 [M+NH₃]⁺.

### compound(6): (1R)-3-(3,4-Dimethoxyphenyl)-1-[3-(t-butoxycarbonylmethoxy)phenyl]-1-propyl (2S)-2-Pip-eridinecarboxylate

A solution of the above Fmoc-protected pipecolyl ester **5** (1.13 g, 1.5 mmol) in CH₂Cl₂ (25 mL) was treated with piperidine (1 mL, 10 mmol) and DBU (1 ml), and the mixture was stirred overnight at ambient temperature. The reaction mixture was concentrated and flash-chromatographed (silica gel, 50 to 100% EtOAc/hexanes) to afford the free amine (694 mg, 90%) as a white sticky foam: **TLC** (MeOH/EtOAc, 1/8) Rf = 0.30, **¹H NMR** (400 MHz, DMSO-d6) 7.22 (t, J = 7.8 Hz, 1 H), 6.98 (d, J = 7.6 Hz, 1 H), 6.91 (s, 1 H), 6.82 (m, 2H), 6.73 (d, J = 8.0 Hz, 1 H), 6.67 (s, 1 H), 5.79 (dd, J = 6.2, 6.6 Hz, 1 H), 4.52 (s, 2H), 3.90 (s, 6H), 3.44 (m, 1 H), 3.32 (s, 1 H), 3.04 (m, 1 H), 2.62-2.32 (m, 3H), 2.28-2.14 (m, 1 H), 2.09-2.03 (m, 1 H), 1.95 (m, 1 H), 1.52 (s, 9H), 1.73-1.53 (m, 4H); **¹³C NMR** (101 MHz, DMSO-d6) 173.8, 168.4, 158.9, 149.1, 147.5, 142.2, 134.7, 129.3, 120.6, 120.2, 114.3, 113.7, 112.0, 111.5, 82.8, 75.3, 66.0, 61.6, 56.5, 56.4, 48.7, 38.4, 31.4, 29.2, 28.3, 25.8, 22.9; **LCMS**(C4, ESI-MS) rt = 7.39 min, *m*/*z* = 513.98 [M+H]⁺_{;}

### compound(7,8): SLF'

A solution of the above amine **6** (165 mg, 0.32 mmol) in CH₂Cl₂ (10 mL) was treated with 2-ethyl-(3,4,5-trimethoxyphenyl)acetic acid (96.5 mg, 0.38 mmol) followed by EDC. HCl (106.4 mg, 0.56 mmol), HOBt (75mg, 0.55 mmol) and diisopropylethylamine (140 µL, 0.8 mmol) under a nitrogen atmosphere at ambient temperature. The resulting solution was allowed to stir overnight. The reaction mixture was then concentrated and flash-chromatographed (silica gel, 20 to 50% EtOAc/hexanes) to afford the penultimate tert-butyl ester protected pipecolylphenylacetamide as a white foam **7** (160 mg, 70%). **LCMS** (C4, ESI-MS) rt = 10.07 min, *m*/*z* = 749.80 [M+H]⁺.
A solution of the tert-butyl ester **7** (150 mg, 0.2 mmol) in CH₂Cl₂ (3 mL) was treated with trifluoroacetic acid (1 mL), and the mixture was stirred at ambient temperature for 3 h. The reaction mixture was then concentrated, and flash-chromatographed (silica gel, EtOAc with 1% acetic acid) to afford the acid as a white solid **8** SLF' (96 mg, 70%). **TLC** (MeOH/EtOAc, 1/5) Rf = 0.15; **¹H NMR** (400 MHz, DMSO-d6) 9.85 (s, 1H), 7.16 (t, J = 8.0Hz, 1 H), 6.84-6.59 (m, 6H), 6.37 (s, 2H), 5.67-5.45 (m, 2H), 4.77-4.49 (m, 2H), 3.77 (s, 3H), 3.76 (s, 3H), 3.75 (s, 3H), 3.74 (s, 6H), 3.69-3.49 (m, 2H), 3.19-3.12 (m, 1 H), 2.61-2.45 (m, 2H), 2.26-1.93 (m, 4H), 1.69-1.44 (m, 4H), 1.40-1.15 (m, 2H), 0.76 (t, J = 7.6Hz, 3H), **¹³C NMR** (101 MHz, DMSO-d6) 172.7, 171.3, 169.5, 156.9, 152.3, 147.8, 146.3, 141.0, 135.9, 134.6, 132.4, 128.7, 119.2, 118.7, 114.0, 110.6, 110.4, 110.1, 103.8, 75.4, 64.2, 59.5, 55.3, 54.9, 54.8, 51.6, 49.9, 42.5, 37.1, 30.4, 28.7, 26.6, 25.8, 23.8, 19.9, 11.5. **LCMS**(C4, ESI-MS) rt = 8.87min, *m*/*z* = 693.88 [M+H]⁺

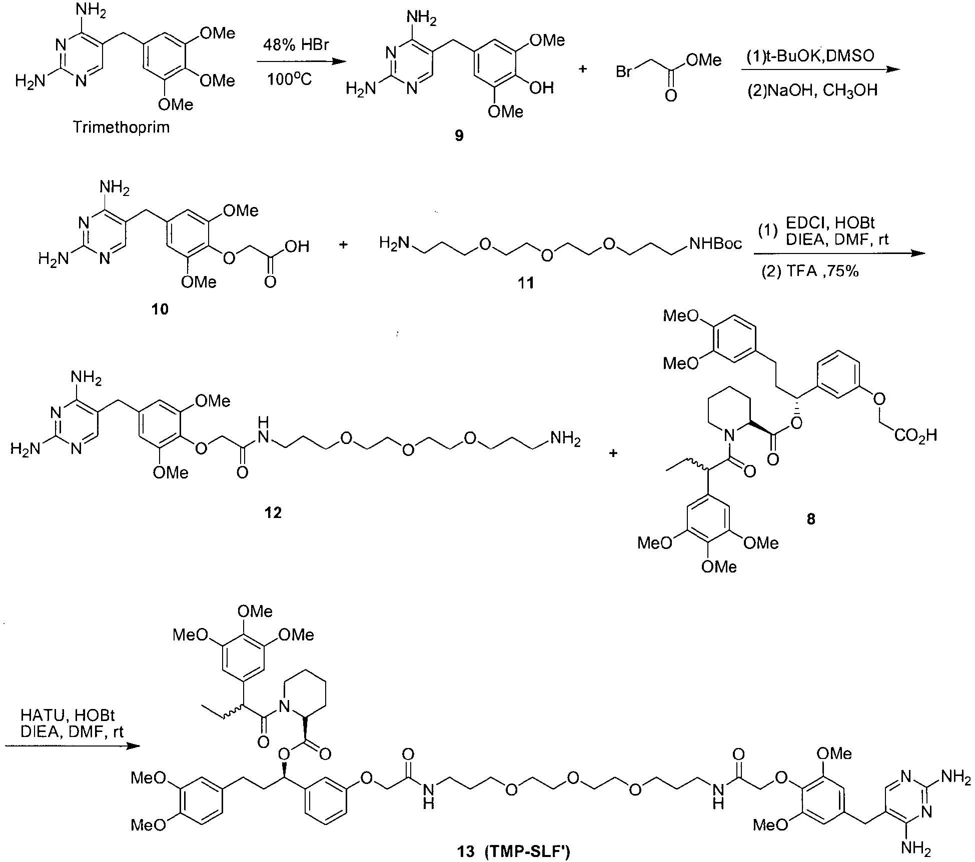

### compound(9): 4-((2,4-Diaminopyrimidin-5-yl)methyl)-2,6-dimethoxyphenol

Trimethoprim (TMP) was converted to a 4'-substituted phenol derivative by preferential cleavage of the 4'-methoxy group. 5 g of TMP was dissolved in 60 mL 48% HBr preheated to ca. 100°C. Reaction was stirred for ca. 20 min and then quenched by slow addition of 12 mL 50% NaOH. Reaction was allowed to cool to room temperature, and placed at 4°C overnight, allowing crystals to form. Crystals were filtered and washed with ice-cold water. Crystals were dissolved in ca. 25 mL boiling water, and the solution was neutralized with NH₄OH, leading to recrystallization. Crystals were filtered and dried under vacuum, yielding the desired phenol 9 (3g, 60%). ¹H NMR (MeOD, 400 MHz), 7.10 (s, 1 H), 6.44 (bs, 2H), 3.71 (s, 6H), 3.53 (s, 2H). LCMS (C4, ESI-MS) rt = 1.60 min, m/z = 277.02 [M+H]⁺_{;}

### compound(10): 2-(4-((2,4-Diaminopyrimidin-5-yl)methyl)-2,6-dimethoxyphenoxy)-acetic acid

To a solution of the 4'-phenol derivative of **9** (2.3 g, 8.3 mmol) in DMSO (28 ml, 300 mM) was added 1.1 equiv. of t-BuOK with stirring under Ar atmosphere. The solution was stirred at room temperature for 0.25-0.5 h. Occasionally, a white precipitate would form during this time. To the solution was added 1.1 equiv. of methyl bromoacetate (9.13 mmol, 1.38 g, 861 µL), and the solution was stirred at room temperature. The reaction was monitored by TLC on silica gel with CH₂Cl₂:CH₃OH (5:1). When completed (within 2 h), the solvent was removed under vacuum, and the residual brown oil was subjected to column chromatography on silica gel, elution with 5-10% CH₃OH/CH₂Cl₂. Fractions containing the desired product were pooled and concentrated to the 4'-substituted ester (1.4 g, 50%). **TLC** (MeOH/EtOAc, 1/5) Rf = 0.6; **LCMS** (C4, ESI-MS) rt = 4.88 min, *m*/*z* = 349.11 [M+H]⁺_{;} To a solution of the ester (1 g, 2.86 mmol) in CH₃OH (15 ml, 200 mM) was added 3.0 equiv. of 1.0 N NaOH. The solution was stirred at room temperature for ca. 1 h, and neutralized with 3.0 equiv. of 1.0 N HCl. This led to precipitation of the hydrolyzed 4'-alkylated TMP derivative, which was then filtered, washed with ice-cold water and dried under vacuum (0.7g, 70%). **¹H NMR** (400 MHz, DMSO-d6) 7.51 (s, 1 H,), 6.56 (s, 2 H), 4.38 (s, 2 H), 3.72 (s, 6 H), 3.53 (s, 2H). **LCMS** (C4, ESI-MS) rt = 3.26 min, *m*/*z* = 335.65 [M+H]⁺_{;}

### compound(11): tert-Butyl 3-(2-(2-(3-aminopropoxy)ethoxy)ethoxy)propylcarbamate

A solution of the 3-(2-(2-(3-aminopropoxy)ethoxy)ethoxy)propan-1-amine (6.8 ml) in CH₂Cl₂ (60 mL) was added (Boc)₂O (3.6 g) in CH₂Cl₂ (240 mL) slowly for 6 h, and then stir under Ar atmosphere overnight. The reaction mixture was concentrated and washed with Na₂CO₃, dried with MgSO₄, and evaporated under vacuum to give the product (4.3 g, 80%). **¹H NMR** (400 MHz, DMSO-d6) 5.15 (s, 1 H), 3.45-3.60 (m, 12H), 3.16 (dd, J = 4.8Hz, 2H), 2.73 (s, 2H), 1.73-1.65 (m, 4H), 1.38 (s, 9H); **¹³C NMR** (101 MHz, DMSO-d6) 156.2, 79.3, 70.8, 70.4, 70.3, 39.8, 33.2, 28.6, **LCMS** (C4, ESI-MS) rt = 5.48min, *m*/*z* = 321.10 [M+H]⁺

### compound(12): N-(3-(2-(2-(3-Aminopropoxy)ethoxy)ethoxy)propyl)-2-(4-((2,4-diaminopyrimidin-5-yl)methyl)-2,6-dimethoxyphenoxy)acetamide

A mixture of 10 (0.64 mmol, 213 mg) and 11 (256 g, 0.8 mmol), HOBt•H₂O (123 mg, 0.80 mmol), EDCl•HCl (153 mg, 0.80 mmol), N,N'-diisopropylethylamine (297 µL, 0.80 mmol) in dry DMF (8 mL) was stirred for 5 h at room temperature. The mixture was diluted with AcOEt and washed with saturated NaHCO₃ (x2) and brine followed by drying over Na₂SO₄. After removal of the solvent *in vacuo* the residue was purified by column chromatography reverse phase HPLC (20-60% H₂O/CH₃CN) to give **12** (258 mg, 60%). **LCMS** (C4, ESI-MS) rt = 5.58 min, *m*/*z* = 637.24 [M+H]⁺. A solution of **12** (200 mg, 0.2 mmol) in CH₂Cl₂ (6mL) was treated with trifluoroacetic acid (2 mL), and the mixture was stirred at ambient temperature for 3 h. The reaction mixture was then concentrated, and afford product **12** (150 mg, 90%). **LCMS** (C4, ESI-MS) rt = 4.52 min, *m*/*z* = 537.26 [M+H]⁺.

### compound(13): TMP-SLF'

A mixture of **12** (0.155 mmol, 83.1 mg) and **8** (100 g, 0.144 mmol), HOBt•H₂O (29.2 mg, 0.216 mmol), HATU (82.1 mg, 0.216 mmol), *N,N'*-diisopropylethylamine (60 µL, 0.36 mmol) in dry DMF (4 mL) was stirred for 8 h at room temperature. The mixture was diluted with AcOEt and washed with saturated NaHCO₃ (x2) and brine followed by drying over Na₂SO₄. After removal of the solvent *in vacuo* the residue was purified by column chromatography reverse phase HPLC (20-60% H₂O/CH₃CN) to give **13 (TMP-SLF')** (60 mg, 40%). **¹H NMR** (400 MHz, DMSO-d6) 7.90 (s, 1 H), 7.12 (m, 1 H), 6.89-6.58 (m, 6H), 6.46 (s, 2H), 6.38 (s, 2H), 5.75-5.52 (m, 2H), 4.50-4.46 (m, 4H), 3.86-3.80 (m, 21 H), 3.65-3.41 (m, 16H), 3.22-3.14 (m, 3H), 2.65-2.47 (m, 4H), 2.28-2.00 (m, 4H), 1.86-1.39 (m, 8H), 1.33-1.25 (m, 2H), 0.86 (t, 3H); **¹³C NMR** (101 MHz, DMSO-d6) 173.3, 171.1, 170.5, 168.7, 164.5, 162.1, 157.6, 155.9, 153.6, 153.1, 149.1, 147.7, 142.4, 141.0, 136.2, 133.5, 132.1, 130.2, 120.4, 120.2, 114.6, 113.1, 112.0, 111.6, 105.7, 105.1, 76.2, 72.6, 70.6, 70.4, 69.6, 69.3, 67.5, 61.1, 56.5, 56.4, 56.2, 56.1, 52.6, 50.9, 43.6, 40.1, 38.4, 37.5, 36.9, 34.0, 31.5, 29.4, 28.2, 26.9, 25.0, 21.2, 12.7; **LCMS** (C4, ESI-MS) rt = 6.59 min, *m*/*z* = 1212.52 [M+H]⁺_{;} **HRMS** (ESI) *m*/*z* calculated for C₆₃H₈₅N₇O₁₇ 1212.60747, found 1212.60982 [M+H]⁺.

### Example 2:

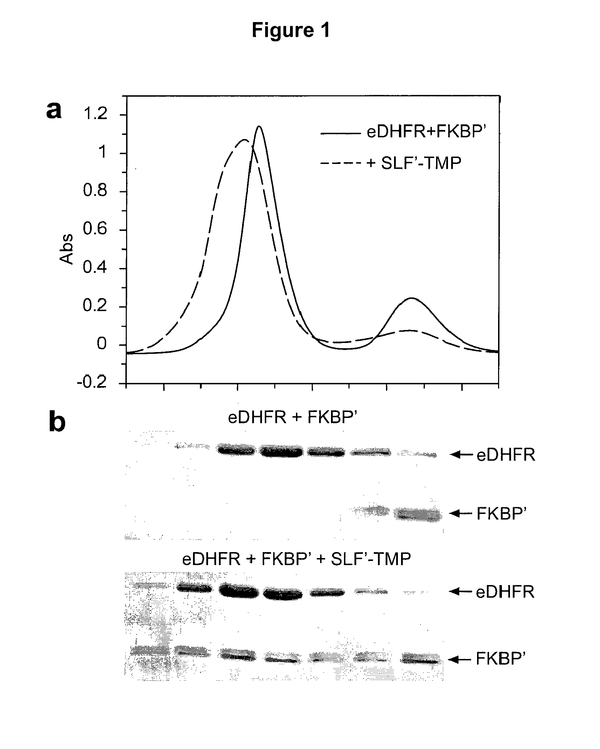

A size exclusion chromatography profile of a mixture of 100 µM eDHFR and FKBP(F36V) was performed in the absence or the presence of 100 µM SLF'-TMP (see Fig. 1a). The absorption curve is significantly shifted to higher molecular weight in the presence of 100 µM SLF'-TMP. This shows that SLF'-TMP binds effectively to FKBP(F36V) and eDHFR, respectively.

### Example 3:

A SDS-PAGE analysis of fractions from the gel filtration was carried out in the absence and the presence of the ligand SLF'-TMP, respectively (see Fig. 1b). The upper panel shows the gel in the absence of SLF'-TMP, the lower panel in the presence of SLF'-TMP. It can be clearly seen by the double bands that SLF'-TMP binds to FKBP(F36V) and eDHFR, respectively.

### Example 4:

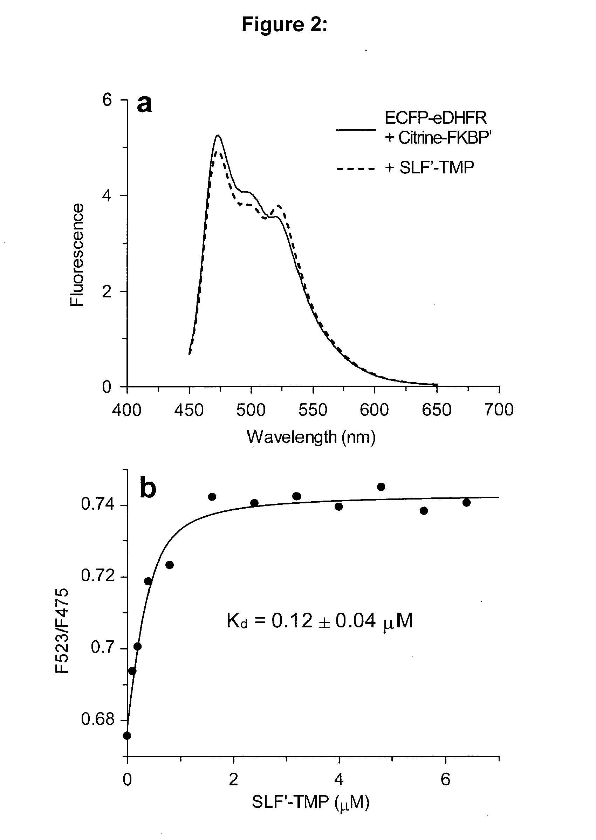

To further confirm the efficiency of ligand-induced heterodimerization the binding domains were fused to fluorescent proteins to produce recombinant ECFP-eDHFR and Citrine-FKBP(F36V). (ECFP = enhance cyan fluorescent protein; YFP = yellow fluorescent protein; herein Citrine, a monomeric yellow fluorescent protein variant, was used.) The fluorescence resonance energy transfer (FRET) between ECFP and Citrine was used to access the ligand-induced heterodimerization. These fluorescent proteins were chosen as fusion protein partners for eDHFR and FKBP(F36V), respectively, because of their easy detectability and discernibility as a FRET pair. Addition of the ligand led to a dose-dependent increase of FRET as judged from the significant decrease of the CFP fluorescence intensity at 475 nm (emission peak of ECFP) and the concomitant increase of the fluorescence intensity at 527 nm (emission peak of Citrine) upon excitation at 370 nm (Figure 2a). Herein, fluorescence spectra of a mixture of 400 nM ECFP-eDHFR and 400 nM YFP-FKBP(F36V) were registered before and after addition of 400 nM SLF'-TMP.

### Example 5:

A titration experiment of ECFP-eDHFR and YFP-FKBP(F36V) with SLF'-TMP was performed. A dose-dependent increase in FRET signal upon titration of SLF'-TMP into a mixture of 400 nM ECFP-eDHFR and 400 nM YFP-FKBP(F36V) was observed. The FRET signal (the ratio of fluorescence intensity at 523 nm versus fluorescence intensity at 475 nm) was plotted against the ligand concentration. The solid line could be fitted to a quadratic equation yielding a dissociation constant K_{d} of 0.12 ± 0.04 µM (see Fig.2b).

### Example 6:

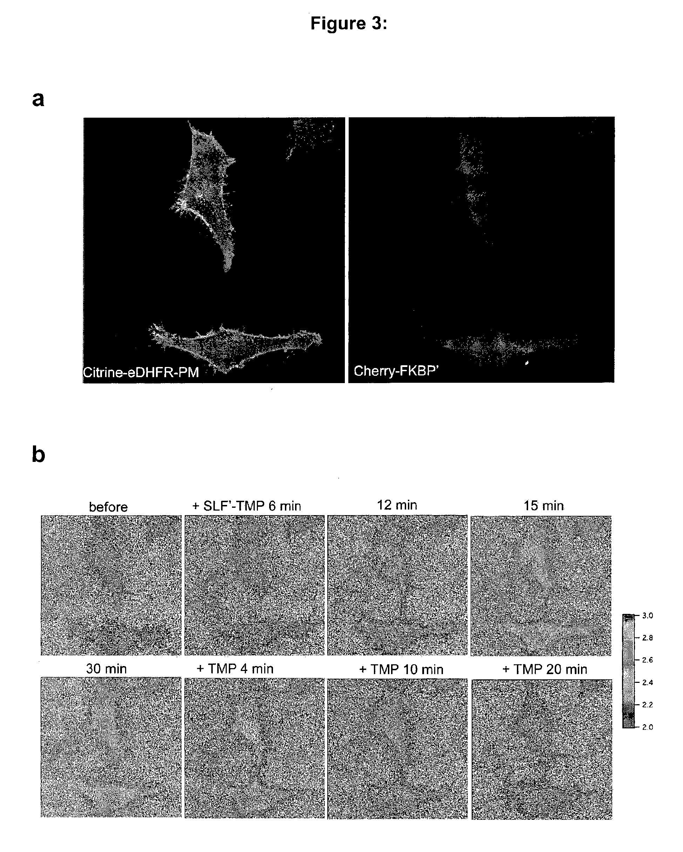

To test the application of the CID system in cells, two domains of FKBP(F36V) and eDHFR were fused N-terminally to a monomeric Cherry variant of red fluorescence protein and Citrine, respectively. A plasma membrane targeting sequence (PM), the K-Ras C-terminus, was placed at the C-terminus of Citrine-eDHFR, resulting in a chimeric construct Citrine-eDHFR-PM. HeLa cells were transfected with both Citrine-eDHFR-PM and Cherry-FKBP(F36V) plasmids. Citrine-eDHFR-PM was largely observed at the plasma membrane, whereas Cherry-FKBP(F36V) was detected everywhere in the cell (see Fig. 3a). The expression in the cells was assessed by FLIM (fluorescence-lifetime imaging microscopy) measurements. Confocal microscopy yielded images of HeLa cells coexpressing Citrine-eDHFR-PM and Cherry-FKBP(F36V).

### Example 7:

SLF'-TMP-induced heterodimerization was measured by FRET between the donor Citrine and the acceptor Cherry with the use of FLIM. FLIM is an imaging technique based on measurement of the lifetime of a fluorophore. When there is a FRET, energy transfer from the donor molecule to the acceptor molecule will decrease the lifetime of the donor, which can be recorded by FLIM. Since the FLIM-based FRET measurements are insensitive to the concentration of fluorophores and can thus filter out artifacts resulting from changes in the concentration and emission intensity, FLIM has been a very useful technique for monitoring protein interactions in cells. Addition of 1 µM SLF'-TMP to the cells co-expressing Citrine-eDHFR-PM and Cherry-FKBP(F36V) led to a rapid decrease of the fluorescence life time of Citrine from ca. 2.95 to ca. 2.72 ns, which was not observed in control cells co-expressing Citrine-eDHFR-PM and Cherry (Fig. 3b, upper panel). These results suggest that the ligand triggered heterodimerization of Citrine-eDHFR-PM with Cherry-FKBP(F36V). Importantly, subsequent supplementation of 10 µM competitor TMP led to a recovery of the fluorescence life time (average ca. 2.91 ns at the steady state) (Fig. 3c), indicating that protein-protein interactions were disrupted.

A rapid dimerization induced by adding SLF'-TMP and a rapid reverse process mediated by TMP were observed (Fig. 3c). Finally, there was no cytotoxicity observed at various concentrations of SLF'-TMP and TMP tested in the experiments (Fig. 5).

### Example 8:

Oscillations of induced protein dimerization can be generated by applying multiple rounds of the dimerizer, SLF'-TMP, and the competitor, TMP. As shown in Figure 4, switches between dimerization "on" and "off" were achieved by adding tandem the ligand and the competitor.

## Claims

1. Compound of the general formula (I):
**SLF' ― X ― TMP**
wherein X is a linker molecule selected from the group comprising polyethylene glycol, triethylene glycol, tetraethylene glycol, alkyl chain with up to 30 carbon atoms, phosphodiesters, glycosides, amides, esters, diesters, thioesters, aldol products, acetate moieties, polyethylenes, isoprenoids, phenyl groups, aromatic groups, hetrocyclic groups, ethers, thioethers, and imines; and TMP represents trimethoprim.

2. Compound according to claim 1, wherein X is TEG.

3. Bioorthogonal system for testing intracellular protein interaction in cells, comprising
a) the compound according to claim 1;
b) fusion protein 1 generated from a test compound 1 and at least the SLF' binding domain of FKBP(F36V); and
c) fusion protein 2 generated from a test compound 2 and at least the TMP binding domain of a bacterial DHFR.

4. Bioorthogonal system according to claim 3,
wherein test compound 1 and test compound 2 are selected independently from one another among gene products, proteins, protein domains, peptides, polypeptides, glycopeptides, proteins with secondarily modified amino acids, peptides or proteins with protecting groups, saccharides, small molecules, lipids, polynucleotides, oligonucleic acids, DNA and RNA.

5. Bioorthogonal system according to claim 3 or 4, wherein the bacterial DHFR is eDHFR.

6. Bioorthogonal system according to any of claims 3 to 5, additionally comprising d) TMP, SLF' or other DHFR or FKBP(F36V) ligands such as MTX

7. Bioorthogonal system according to any of claims 3 to 6, wherein X in SLF' ― X ― TMP is TEG.

8. Use of the bioorthogonal system as defined in any of claims 3 to 7 for testing the interactions of a test compound 1 with a test compound 2.

9. Method for testing intracellular protein interaction in cells, comprising the following steps:
a) Providing, transfecting and expressing the DNA sequence of a fusion protein 1 from a test compound 1 to be tested and at least the SLF' binding domain of FKBP(F36V);
b) providing, transfecting and expressing the DNA sequence of a fusion protein 2 from a test compound 2 to be tested and at least the TMP binding domain of a bacterial DHFR;
c) adding SLF' ― X ― TMP according to claim 1 or 2 to cells and letting them pass the plasma membrane; and
d) measuring the physiological effect by determining the change in a selected test parameter system.

10. Method according to claim 9, wherein the generated effect is reverted by contacting cells with TMP, SLF' or other DHFR or FKBP(F36V) ligands such as MTX in a concentration that allows for competitive replacement of SLF' ― X ― TMP from a bacterial DHFR binding site.

11. Method according to claim 9 or 10,
wherein test compound 1 and test compound 2 are selected independently from one another among gene products, proteins, protein domains, peptides, polypeptides, glycopeptides, proteins with secondarily modified amino acids, peptides or proteins with protecting groups, saccharides, small molecules, lipids, polynucleotides, oligonucleic acids, DNA and RNA.

12. Method according to any of claims 9 to 11, wherein the bacterial DHFR is eDHFR.

13. Method according to claim 11, wherein test compound 1 and test compound 2 is a physiological peptidic compound, respectively.

14. Method according to claim 11, wherein test compound 1 is a pharmaceutical drug and test compound 2 is a physiological peptidic compound, or test compound 1 is a physiological peptidic compound and test compound 2 is a pharmaceutical drug.

15. Kit, comprising
a) SLF' ― X ― TMP,
b) TMP, SLF' or other DHFR or FKBP(F36V) ligands such as MTX, and
c) the nucleotide sequences or the vectors including the nucleotide sequences coding for at least the binding domains of FKBP(F36V) and a bacterial DHFR.
